# EUROPEAN PATENT SPECIFICATION

(11) **EP 4 608 262 B1**
(45) Date of publication and mention of the grant of the patent: **22.07.2026**
(21) Application number: 23801882.4
(22) Date of filing: 25.10.2023
(51) Int. Cl.: A61B 5/15, A61B 5/151

(54) **BLOOD SAMPLER FOR COLLECTING A BLOOD SAMPLE FROM A DIGIT**
BLUTENTNAHMEGERÄT ZUR ENTNAHME EINER BLUTPROBE AUS EINEM ZIFFERNTEIL
ÉCHANTILLONNEUR DE SANG POUR COLLECTER UN ÉCHANTILLON DE SANG À PARTIR D'UN DOIGT

(30) Priority: 25.10.2022 BE 202205866
(43) Date of publication of application: 03.09.2025
(73) Proprietor: Voxdale BV, 2110 Wijnegem (BE); Instituut Voor Tropische Geneeskunde, 2000 Antwerpen (BE)
(72) Inventor: WYMEERSCH, Rien, 9000 Gent (BE); DIERYCKX, Tim, 9030 Gent (BE); SORGELOOS, Kristof, 9120 Beveren-Waas (BE); DE BAETSELIER, Irith, 2160 Wommelgem (BE); PLATTEAU, Tom, 2650 Edegem (BE); HERRIJGERS, Corinne, 2060 Antwerpen (BE); VAN DEN BOSSCHE, Dorien, 1851 Humbeek (BE)
(74) Representative: Winger
(86) International application number: PCT/IB2023/060764
(87) International publication number: WO 2024/089619

(56) References cited:
- WO-A1-2018/039307
- WO-A1-2020/167746
- CN-A- 114 081 488
- US-A1- 2009 198 152
- US-A1- 2012 271 197

## Description

### Technical field of the invention

The present invention relates to the field of blood samplers. More specifically, the present invention describes a blood sampler for collecting a blood sample from a digit, and a kit of parts comprising the blood sampler.

### Background of the invention

Blood samplers for collecting a blood sample from a digit, i.e., a finger or a toe, that may be used by untrained persons, are known in the art. An advantage of such blood samplers is that they can be used for taking a blood sample by the person from whom the blood sample is to be taken.

The process for self-collecting of a blood sample may be divided into two phases: a preparatory phase and a needle puncturing and collection phase. In the state of the art, the preparatory phase may be used to optimize the blood flow to facilitate the steps to follow. In already existing self-collection tests, people are asked to first wash their hands with warm water, which is followed by a hand massage. Afterwards, a finger from which the blood sample is to be collected may be disinfected, for instance with an alcohol wipe. A ring finger or index finger is preferred. The needle puncturing and collection phase commences when the finger is lanced by a lancet, e.g., needle. Any released blood may be collected with a capillary, which is then emptied into a tube that is later sent to a laboratory. Alternatively, the finger is punctured and blood is simply allowed to leak into a tube. The latter is referred to as "free fall collection". Both methods typically require massaging to get enough blood released. Once the tube is full, it can be sent for further processing. After collection, a plaster is placed around the finger to prevent any contamination.

An example of a blood sampler of the state of the art is disclosed in WO2020/245258, which describes a blood sampler comprising an attachment ring for fixing the blood sampler with respect to a finger. Thereby, the finger is brought in position with respect to an opening in the blood sampler. A lancet is movable in the opening between a retracted position, and a lancing position wherein the lancet extends at least partially in the space for receiving the finger. After moving the lancet in the lancing position, thereby lancing the finger, blood may be collected through the opening, into a vial. By the design of the blood sampler, the vial may have to be a dedicated vial, especially designed for this particular blood sampler. This limits the types of vials that could be used, which may cause a problem when the blood sampler is to be produced and used at a cost as low as possible. This problem may be particularly important in low income countries. Furthermore, the blood sampler may be difficult to use when lancing at multiple locations (on the same finger, or on different fingers), whenever collecting a large amount of blood is needed. Indeed, transfer of blood from the vial to a secondary vial may be needed before the lancet may be used again for lancing at a further location. Further, WO 2020/167746 A1 discloses an integrated finger-based capillary blood collection device with the ability to lance and squeeze a finger, wherein the device comprises a finger receiving portion and a single opening for receiving a lancet housing and a collection container.

There is thus still a need in the art for devices that address at least some of the above problems.

### Summary of the invention

It is an object of the present invention to provide a good blood sampler. It is a further object of the present invention to provide a good kit of parts comprising said blood sampler.

The above objective of the present invention is accomplished by a blood sampler according to claim 1. Preferred embodiments of the present invention are defined in the dependent claims.

It is an advantage of embodiments of the present invention that standard vials may be used for the blood sampler. It is a further advantage of embodiments of the present invention that costs of manufacturing and/or using the blood sampler may be small.

It is an advantage of embodiments of the present invention that a single vial may be used for collecting blood from different lanced locations on a digit.

It is an advantage of embodiments of the present invention that the blood sampler may have a relatively simple design.

It is an advantage of embodiments of the present invention that the blood sampler may allow for collecting blood without squeezing or massaging the digit. It is generally known that squeezing or massaging the digit deteriorates blood quality.

It is an advantage of embodiments of the present invention that spilling of blood may be limited or even absent, which may result in an efficient collection of the blood sample, and in less anxiety experienced by persons having an anxiety for blood.

In a first aspect, the present invention relates to a blood sampler according to claim for collecting a blood sample from a digit, i.e., from a finger or a toe. The blood sampler comprises a clip defining a space for receiving at least part of the digit. The clip comprises a first opening for attaching a vial for receiving the blood sample. The blood sampler further comprises at least one lancet holder in or secured to the clip at a location different from the first opening. The at least one lancet holder is configured for receiving a lancet system comprising a lancet for lancing the at least part of the digit when present in the space.

In embodiments, the at least one lancet holder comprises at least one second opening in the clip, different from the first opening, for allowing passage of at least a lancet of a lancet system therethrough. It is an advantage of these embodiments that, by having the second opening through which the lancet of the lancet system may pass, integration of the lancet system in the blood sampler may be straightforward.

In embodiments of the present invention, the blood sampler furthermore comprises at least one lancet system comprising a lancet that is movable between a retracted position, and a lancing position wherein the lancet extends at least partially in the space so as to lance the at least part of the digit when present in the space.

In the retracted position, the lancet is preferably not in a position for lancing of the part of the digit, when said part of the digit is located in the space. In embodiments, in the retracted position, the lancet is substantially retracted from the space. In embodiments, the lancet system comprises a lancet casing comprising an interior and an inlet, wherein, in the retracted position, the lancet is substantially located in the interior, and wherein, in the lancing position, the lancet extends at least partially, through said inlet, outside of the interior. It is an advantage of these embodiments that a relatively simple blood sampler configuration is conceived that is relatively simple to manufacture. It is a further advantage of these embodiments that, in the retracted position, i.e., when the lancet is not in use for lancing the at least part of the digit, the lancet is safely located in the interior. This may prevent harm to a person using the blood sampler (e.g. needle stick incident), and may further prevent contamination of the lancet.

In embodiments of the present invention, the lancet system may be a separate, replaceable part of the blood sampler. The lancet system can be sold separately, and connected to or introduced into the lancet holder to form part thereof. In alternative embodiments, the lancet system is integrally made with the other parts of the lancet holder, or forms the lancet holder.

In embodiments, the clip comprises a static portion comprising the first opening, and a movable portion hingeably attached to the static portion. The static portion and/or the movable portion comprises the at least one lancet holder. The at least part of the digit may be located, e.g., clamped, between the static portion and the movable portion. It is an advantage of embodiments having both the first opening and a lancet holder at the static portion that, by having both the first opening and the location at which the lancet system is secured to the clip in the same part of the clip, i.e., in the static part, a spacing between the first opening and said location is independent of a size of the at least part of the digit.

In embodiments, the clip defines a cup peripheral to the space, wherein the first opening is located in a wall of the cup and the at least one lancet holder is located in or at a wall of the cup. The cup may allow for blood to flow from the lanced location of the digit, via the cup, to the vial, attached to the first opening. Furthermore, the cup may result in a spacing between the lanced portion of the digit and the clip, so that the blood may fall freely, without touching the clip, from the digit, through the first opening, into the vial. The lancet holder being located at a wall of the cup may mean that a lancet of the lancet system may pass through a second opening located in a wall of the cup.

In embodiments, an angle between a first direction, from a centre of the space, in absence of the at least part of the digit, to the first opening, and a second direction, from said centre to the lancet system, is at most 70°, such as from 10° to 70°, e.g., from 20° to 70°, for example, from 30° to 60°. Although also larger angles are possible, it is an advantage of these embodiments that the distance between where the at least part of the digit is to be lanced, and the location at which the blood sample is to be collected, may be small. For example, the location where the at least part of the digit is lanced, and the location of the first opening, may be at the same side of the at least part of the digit. This may result in a good efficiency of blood collection, and may prevent blood from first flowing over the at least part of the digit, before it drops into the first opening or into the cup, when present. This may result in efficient collection of the blood sample, and may prevent contamination of the blood sample.

In alternative embodiments, the clip defines a cup and a movable portion, movable, e.g. hingeable, with respect to the cup. Both the cup and the movable portion are peripheral to the space intended for receiving at least part of the digit. The first opening for attaching a vial for receiving the blood sample is located in a wall of the cup, and the at least one lancet holder is located in or at a wall of the movable portion.

In embodiments, the at least one lancet holder may be integrally made with the clip. The lancet holder may for instance be a hole in the cup or in the movable part, for receiving a lancet system. Alternatively, or on top thereof, the lancet holder may comprise a housing, secured to or integrally made with the cup or the movable part, for receiving a lancet system. The housing can have dimensions selected such that the lancet system can be slidingly introduced and snuggly fits therein.

In embodiments, the clip may comprise a friction element such as a rough surface for contacting the at least part of the digit in the space. In embodiments, the clip comprises a clip foam for contacting the at least part of the digit in the space. It is an advantage of these embodiments shifting of the at least part of the digit in the space may be prevented.

In embodiments, the blood sampler comprises means for attaching of the vial to the first opening in any suitable way, for example by a push-fit, e.g., an interference fit or a friction fit, connection. Alternatively, a snap-fit and/or screw connection could be used. A push-fit connection allows for rapid fastening and easy removal of the vial. Typically, the vial, when present, has corresponding means for attaching the vial to the first opening of the blood sampler. In embodiments, the blood sampler comprises the vial. However, this is not essential, and the vial may be only obtained when the blood sampler is used.

In preferred embodiments, the digit is a finger. Preferably, the at least part of the digit that is to be received in the space is at least part of a tip, i.e., at least part of a distal part, of the finger. However, the invention is not limited thereto, and instead, the at least part of the digit that is to be received in the space may be at least part of a middle part or a proximal part of the finger.

In a second aspect, the present invention relates to a kit of parts according to claim 12. The kits of parts comprises the blood sampler according to embodiments of the first aspect of the present invention. Furthermore, the kit of parts comprises a tourniquet for applying a pressure to the digit so as to limit, e.g., stop, a flow of blood through a vein in the digit. Typically, the tourniquet, when tightly wound around the digit for said applying of the pressure to the digit, typically at a location between the part of the digit to be lanced and the palm of the hand, may not limit blood from flowing into the digit, through a vein in a direction from the heart to the digit, but may limit, e.g., substantially hinder or even stop, blood from flowing through a vein from the digit to the heart. As blood may be limited from flowing from the digit to the heart, when the digit has been lanced, the blood may be induced to flow, through the lanced portion of the digit, out of the digit. It is an advantage of these embodiments that a good blood sample may be obtained. It is an advantage of these embodiments that no massaging of the digit may be required for collecting the blood sample. Said massaging may result in a low quality of a collected sample: for example, the massaging may result in the release of particles from walls of the veins which may subsequently be collected in the vial. Moreover, massaging accelerates blood clotting.

In embodiments, the kit of parts furthermore comprises at least one lancet system comprising a lancet that is movable between a retracted position, and a lancing position.

In embodiments, the kit of parts comprises, besides the sampler, also a vial, and a cap for the vial. It is an advantage that the vial may be a standard, commercially available vial. It is an advantage of embodiments of the present invention that no dedicated vial may have to be designed. Therefore, the present invention may result in a reduction of the costs required for manufacturing and using of the kit of parts. However, this is not essential, and the vial may be a dedicated vial.

The vial is typically removably attachable to the first opening, so that the vial may be attached to the first opening shortly before collecting the blood sample, and may be removed after said collecting the blood sample. Preferably, before use of the kit of parts, the vial of the kit of parts is closed by closing means, e.g., by the cap, by a disposable cap, or by a foil. Said closing means may be removed shortly before use of the kit of parts, e.g., shortly before attaching the vial to the first opening. The closing means may prevent contamination of an interior for receiving said blood sample of the vial. After the blood sample has been received in the vial, the vial may be detached from the first opening, and the vial may be closed by the cap.

In embodiments, the vial comprises a screw thread and the cap comprises a complementary screw thread. It is an advantage of embodiments of the present invention that the cap may be screwed on the vial for closing the vial, which may result in a very robust closing of the vial. Alternatively, the vial and the cap may comprise complementary means for obtaining a push-fit or snap-fit connection between the vial and the cap.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG. 1 is a schematic representation of a side view of a blood sampler in accordance with embodiments of the present invention.
FIG. 2 is a cross-sectional view of a blood sampler in accordance with embodiments of the present invention.
FIG. 3 is a schematic representation of a front view of the blood sampler in accordance with embodiments of the present invention.
FIG. 4 is a schematic representation of a perspective view of the blood sampler in accordance with embodiments of the present invention.
FIG. 5 is a schematic representation of a side view of the blood sampler in accordance with embodiments of the present invention, wherein a protective cap has been removed and wherein a lance of the blood sampler is in a retracted position.
FIG. 6 is a schematic representation of a side view of the blood sampler in accordance with embodiments of the present invention, wherein a protective cap has been removed and wherein a lance of the blood sampler is in a lanced position.
FIG. 7 is a schematic representation of the blood sampler in accordance with embodiments of the present invention, when used for collecting a blood sample.
FIG. 8 is a schematic representation of the blood sampler in accordance with embodiments of the present invention, after collection of a blood sample.
FIG. 9 a schematic representation of an exploded view of the blood sampler in accordance with embodiments of the present invention.
FIG. 10 is a schematic representation of a side view of a blood sampler in accordance with embodiments of the present invention, comprising two lancet systems.
FIG. 11 is a schematic representation of a blood sampler in accordance with alternative embodiments of the present invention, wherein a lancet holder is integrally made with a movable part of the clip, and a lancet system is introduced into the lancet holder.
FIG. 12 is an exploded view of the blood sampler of FIG. 11, including the lancet system.
FIG. 13 is a cross-sectional view of the blood sampler of FIG. 11, in which a lancet system is present in the lancet holder.
FIG. 14 is a cross-sectional view of the blood sampler of FIG. 11, without presence of a lancet system.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. The word "comprising" according to the invention therefore also includes as one embodiment that no further components are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled" should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

In a first aspect, the present invention relates to a blood sampler for collecting a blood sample from a digit, i.e., from a finger or a toe. The blood sampler comprises a clip defining a space for receiving at least part of the digit. The clip comprises a first opening for attaching a vial for receiving the blood sample. The blood sampler further comprises at least one lancet holder in or on the clip at a location different from the first opening. The lancet holder is intended for, and configured for, receiving a lancet system comprising a lancet for lancing the at least part of the digit when present in the space. In embodiments, the lancet is movable between a retracted position, and a lancing position wherein the lancet extends at least partially in the space so as to lance the at least part of the digit when present in the space.

In a second aspect, the present invention relates to a kit of parts. The kits of parts comprises the blood sampler according to embodiments of the first aspect of the present invention. Furthermore, the kit of parts comprises a tourniquet for applying a pressure to the digit so as to limit a flow of blood through a vein in the digit.

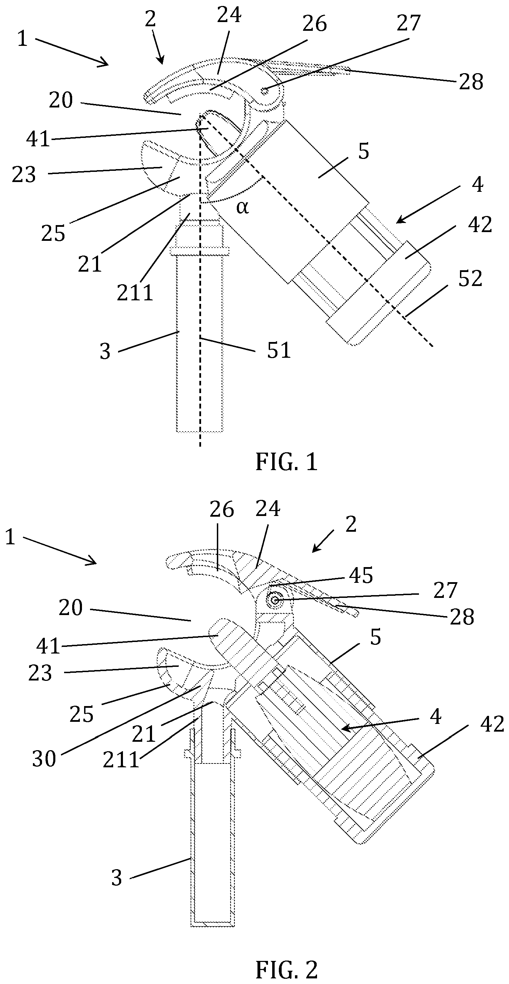

Reference is made to FIG. 1 and FIG. 2. In this example, the blood sampler 1 comprises a clip 2 comprising a static portion 25 and a movable portion 24 hingeably attached to the static portion 25 by hinging means, for instance a pin 27. The clip 2 defines a space 20 for receiving at least part of a digit from a user. The movable portion 24 may be induced to rotate around the hinging means, e.g. the pin 27, so as to increase the size of the space 20 - in the side-view of FIG. 1, said rotation is in a clockwise direction. Thereby, the at least part of the digit may be easily received in said space 20. Inducing the hingeable movement may be facilitated by a lever 28 projecting from the movable portion 24 of the clip. Herein, the user may apply a force to the lever 28 - in the side-view of FIG. 1, a downward force -, for inducing said clockwise rotation.

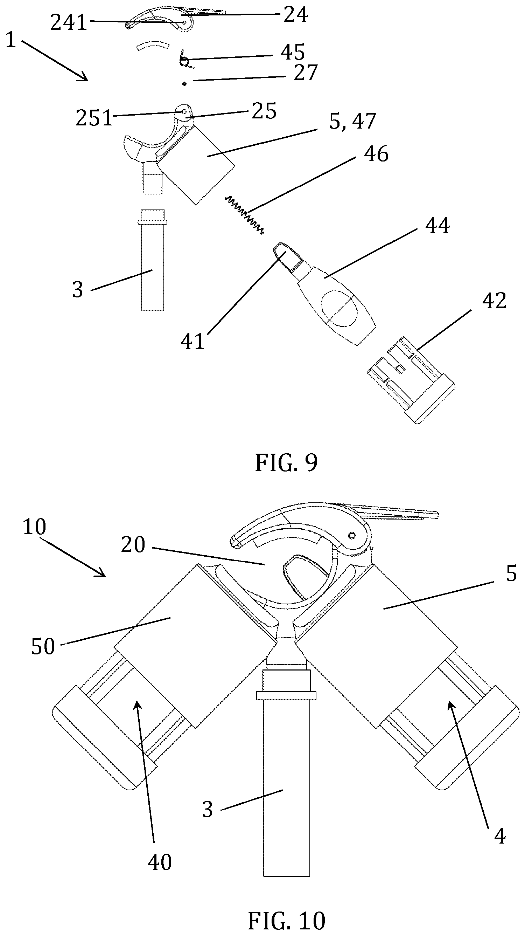

After receiving the at least part of the digit in the space 20, the movable portion 24 may be induced to rotate around the hinging means, e.g. the pin 27, so as to move the static portion 25 and the movable portion 24 towards each other, thereby reducing the size, e.g., volume, of the space 20 - in the side-view of FIG. 1, said rotation is in a counter-clockwise direction. Said counter-clockwise direction may be induced by a clip spring (45, see FIG. 2 and FIG. 9). Thereby, the at least part of the digit may be clamped by the clip 2, between the movable portion 24 and the static portion 25, i.e., the clip 2 may be fastened to the at least part of the digit, such that the at least part of the digit is in a position for being lanced by a lancet of the lancet system 4 introduced in a lancet holder 5 secured to the clip 2. In the embodiment illustrated in FIG. 1 and FIG. 2, the lancet holder 5 and the static portion 25 of the clip 2 are integrally made. A friction element, e.g. a clip foam 26, fixed to the movable portion 24 of the clip 2, may prevent any movement, such as sliding, of the at least part of the digit within the space 20.

In this example, the blood sampler 1 comprises a cup 23 peripheral to the space 20. The cup 23 is located in, e.g., defined by, the static portion 25 of the clip 2. In this example, the blood sampler 1 has a vial 3 attached to a first opening 21, wherein the first opening 21 is located in a wall of the cup 23. In this example, the blood sampler 1 comprises means for said attaching of the vial 3 to the first opening 21 by a push-fit connection. In this example, said means comprises a protruding, substantially cylindrical, portion 211, preferably having an outer diameter that is substantially the same as an inner diameter of an inlet of the vial 3. Thereby, a push-fit, e.g., friction fit, connection may be possible between the vial 3 and the cylindrical portion 211. It is an advantage of these embodiments that any vial having an inlet having an inner diameter substantially the same as an the outer diameter of the protruding portion could be used. Alternatively, an inner diameter of the cylindrical portion 211 may be substantially the same as an outer diameter of the inlet of the vial 3. Instead of a substantially cylindrical shape, the protruding portion could substantially have a truncated conical shape, with a diameter slightly reducing towards an end at which the vial 3 is to be connected, which may facilitate the push-fit connection. Furthermore, the protruding portion may have any other suitable shape: for example, a cross-section of the protruding portion may have a square, triangular, or any other regular or irregular inner circumference, and the vial 3 may have a complementary outer circumference enabling the push-fit.

The lancet system 4 is introduced into a lancet holder 5 located at, for instance made in, secured to or integrally made with, a wall of the cup 23. In the example illustrated, the lancet system 4 is introduced into a lancet holder 5 formed by a tubular element 47 integrally made with the wall of the cup 23. A second opening in the cup 23 allows a lancet of the lancet system 4 to pass through the wall of the cup 23 and into the space 20. In FIG. 1 and FIG. 2, the blood sampler 1 is shown in a preferred position for use, that is, with the cup 23 located directly beneath the space 20. In this position, the first opening 21 is preferably located below the space 20, for example, substantially at a lowest section of the cup 23. An angle α between a first direction from a centre of the space 20, in absence of the at least part of the digit, to the first opening 21, i.e., along dashed line 51, and a second direction, from said centre to the lancet holder 5, i.e., along dashed line 52, is, in this example, about 45°. Preferably, the lancet is oriented with its longitudinal axis substantially along the second direction, and movable substantially along the second direction, i.e., along dashed line 52. As such, in use, the lancet system 4 is introduced into the lancet holder 5 and positioned so that the portion of the at least part of the digit that is lanced may be located above or almost above the first opening 21, so that any blood flowing out of the at least part of the digit may directly drop into the vial 3 attached to the first opening 21 and/or may first drop into the cup 23, and, subsequently, flow into the vial 3. As illustrated in FIG. 2, a lip 30 may be provided at the inside of the cup 23 for faster guiding of drops of blood towards the vial 3. Preferably, the first opening 21 and the vial 3 are adapted for allowing for free-falling, e.g., transfer by gravity, of the blood sample into the vial 3. This may allow for a swift collection of large blood samples, which may be more difficult when, instead, low-volume capillaries, using capillary force, are used to collect the blood sample.

The blood sampler 1 comprises the lancet holder 5, for receiving a lancet system 4, secured to the clip 2 at a location different from the first opening 21. In other words, the location at which blood is collected (i.e., the first opening 21) and the location at which, in use, the lancet enters the space 20, do not overlap. This allows for a much simpler design than when the lancet and the location at which the blood is collected are to be located at substantially the same location. The lancet of the lancet system 4 is movable between a retracted position, wherein the lancet is typically substantially retracted from the space 20, and a lancing position wherein the lancet extends at least partially in the space 20 so as to lance the at least part of the digit when present in the space 20. Before use, the lancet is typically in the retracted position. To further prevent accidental lancing of the user or any other object by the lancet, as well as contamination of the lancet, a protective cap 41 may prevent preliminary exposure of the lancet. For example, the protective cap 41 may cover the lancet. As another example, in embodiments wherein lancet system 4 comprises a lancet casing comprising an interior and an inlet, wherein, in the retracted position, the lancet is substantially located in the interior, and wherein, in the lancing position, the lancet extends at least partially, through said inlet, outside of the interior, the protective cap 41 may cover an inlet of the lancet system 4 so that the lancet may either leave an interior of the lancet system but is then still covered by the protective cap 41, or may block the inlet so that the lancet is altogether prevented from leaving the interior. The protective cap 41 may be removed prior to using the blood sampler for collecting the blood sample, for example, shortly before introducing the at least part of the digit into the space 20. By activating an actuation mechanism, e.g. pushing a knob 42 or engaging a slider (not illustrated), the lancet may be induced to move from the retracted position to the lancing position.

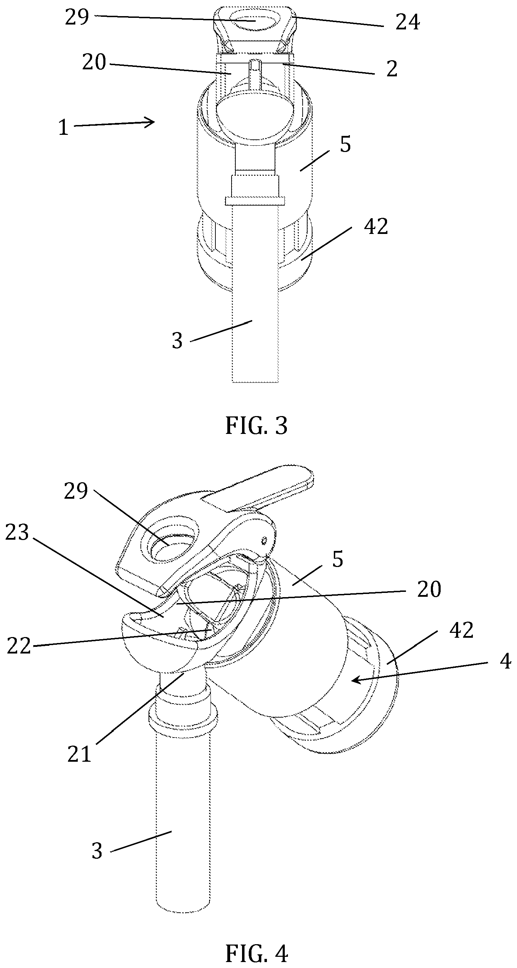

Reference is made to FIG. 3, which is a front view of the blood sampler 1 of FIG. 1. The blood sampler 1 preferably comprises means for correctly locating the at least part of the digit in the space 20. In this example, the movable portion 24 of the clip 2 comprises an aperture 29, so that location and orientation of the at least part of the digit can be determined accurately with respect to the space 20. For example, the aperture 29 may be shaped such that when a nail of a finger overlaps with the aperture 29, the at least part of the finger is correctly located in the space 20, such that the lancet may lance the finger and blood may be efficiently collected from the finger.

Reference is made to FIG. 4, which is a perspective view of the blood sampler 1 of FIGs. 1, 2 and 3. In this example, the clip 2 comprises a second opening 22, different from the first opening 21, wherein the second opening 22 is located in a wall of the cup 23. Herein, the lancet system 4 is introduced in the lancet holder 5 and at the location of the second opening 22, such that the lance of the lancet system 4 is movable through the second opening 22, into the space 20.

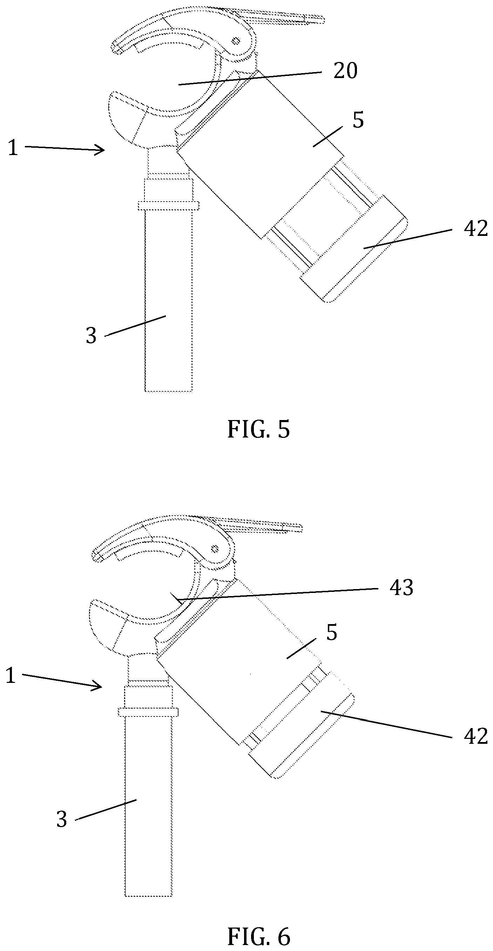

Reference is made to FIG. 5. After removing the protective cap (41, cfr. FIG. 1), the blood sampler 1 may be activated for use. In FIG. 5, the lancet is in the retracted position. In this example, this means that the lancet is not located in the space 20.

By activating the actuation mechanism, e.g. pushing the knob 42, the lancet is induced to move into the lancing position. Reference is made to FIG. 6, where the blood sampler 1 is shown with the lancet 43 in the lancing position, wherein the lancet 43 extends at least partially in the space 20 so as to lance the at least part of the digit when present in the space 20.

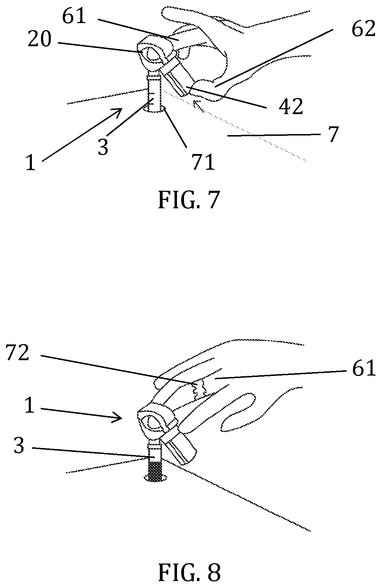

Reference is made to FIG. 7. A part of the tip, i.e., a distal part, of a middle finger 61 of the user is received in the space 20 of the blood sampler 1. In this example, the blood sampler 1 is configured so that, when the tip of the middle finger 61 is located in the space 20, the actuation mechanism may be activated, e.g. the knob 42 may be pushed by a thumb 62 of the same hand that comprises the middle finger 61 of the user. This may allow for a reproducible lancing of the digit, e.g., middle finger 61, reproducible at a predetermined location on the digit, whenever the thumb 62 is used for pushing the knob 42. By pressing the knob 42, the lancet is moved into a lancing position, and lances the middle finger 61 of the user.

In this example, a kit of parts in accordance with embodiments of the present invention further comprises a box 7 comprising an aperture 71, wherein the vial 3 may be settled. When the box 7 is located on a table, by settling the vial 3 into the aperture 71, it may be ensured that the blood sampler 1 is located at a height below that of the heart, so that gravity may induce the blood to flow from the lanced portion of the digit, without requiring massaging of the digit.

Reference is made to FIG. 8. The kit of parts further comprises a tourniquet 72, that may be fixed around the finger to be pierced, e.g. the middle finger 61. The tourniquet is, preferably, fixed around the middle or proximal part of the finger to be pierced, e.g. middle finger 61, before moving the tip of that finger 61 in the space 20 of the blood sampler 1. The tourniquet 72 applies a pressure to veins in the finger 61 that are for flowing blood in a direction from the finger 61, back to the heart of the user. By blocking said flow of the blood, the blood may be induced to flow through the lanced portion of the finger 61, and may be collected by the vial 3. However, the tourniquet 72 may not be essential when using the blood sampler 1 for collecting the blood sample.

Reference is made to FIG. 9, which is an exploded view of the blood sampler 1 provided with a lancet system 4. The lancet system 4 may comprise a lancet casing 44, which may comprise an interior and an inlet, wherein the inlet is covered by the protective cap 41, wherein, in the retracted position, the lancet 43 is substantially located in the interior. In this example, the lancet casing 44 is substantially located in a cavity defined by a knob 42 and lancet holder 5 under the form of a tubular element 47, wherein the outer diameter of the knob 42 is substantially the same as an inner diameter of the tubular element 47 forming the lancet holder 5, such that the knob 42 may be slid into the tubular element 47. When pushing the knob 42, the lancet casing 44 may be induced to release the lancet 43 from said interior, such that the lancet extends at least partially, through said inlet, outside of the interior, thereby moving the lancet into the lancing position.

Alternatively, the lancet is fixed with respect to the lancet casing 44 such that at least part of the lancet projects from the lancet casing 44. Said protective cap 41 may cover the lancet, so that removing the protective cap 41 may expose the lancet. The blood sampler 1 may further comprise one or more springs 46 located between the knob 42 and the lancet holder 5, in the embodiment illustrated formed by the tubular element 47, secured to, e.g. integrally made with, the clip 2. When pushing the knob 42, the lancet casing 44 may move towards the space, such that the lancet moves into the space, thereby compressing the spring 46. When the knob 42 is subsequently released, the spring 46 decompresses, so that the lancet casing 44 is pushed away from the space such that the lancet moves out of the space.

The static portion 25 of the clip comprises an aperture 251 and the movable portion 24 comprises an aperture 241, wherein said apertures 241 and 251 can be oriented such that a hinging means, e.g. pin 27, can be received simultaneously by both apertures 241 and 251 so as to form a hingeable connection between the static portion 25 and the movable portion 24. A clip spring 45 is provided for applying a force for moving the movable portion 24 and the static portion 25 towards each other for reducing a size of the space 20 defined by the movable portion 24 and the static portion 25.

Reference is made to FIG. 10. The blood sampler 1 in accordance with embodiments of the present invention is not limited to a single lancet holder 5 as described above, but may, instead, comprise a plurality of lancet holders 5, for each receiving a lancet system 4. In FIG. 10, the blood sampler 10 comprises the lancet holder 5, illustrated with a lancet system 4 introduced therein, and a further lancet holder 50, illustrated with a further lancet system 40 introduced therein. The further lancet holder 50 may have substantially the same features as the lancet holder 5, as set out above. It is an advantage of having the further lancet holder 50 that the at least part of the digit, when located in the space 20, may be lanced at two different locations at the same time or subsequently, without having to move the digit, which may result in an efficient collection of a blood sample.

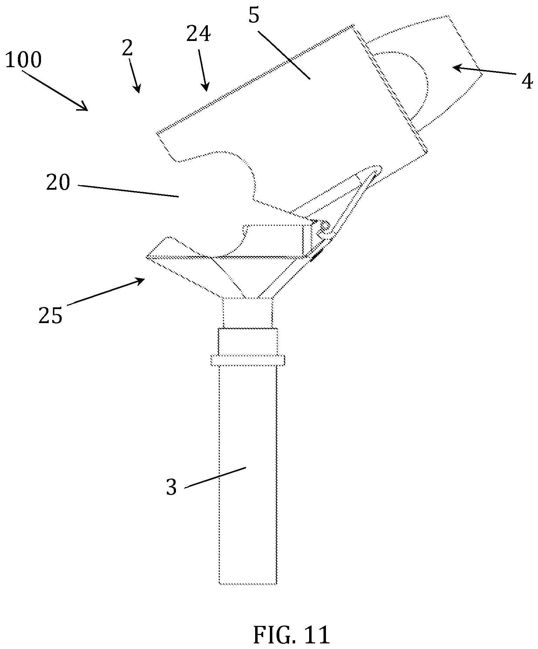

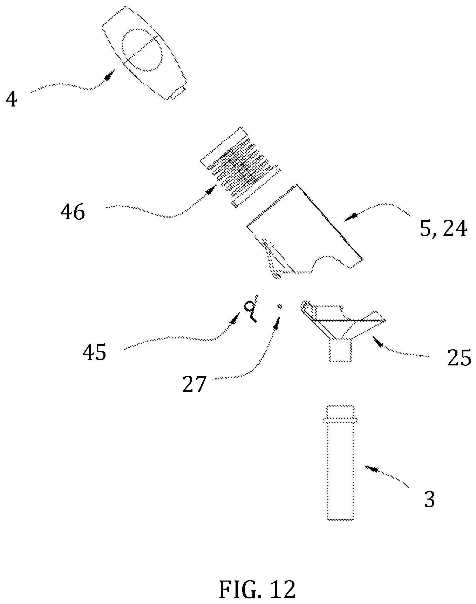

FIG. 11 to FIG. 14 illustrate another embodiment of a blood sampler 100 according to the present invention. In order not to enter into repetition, not all details of these drawings are described in as much detail as the for the previous drawings. However, device features with same reference numbers are same or similar to the device features with corresponding reference numbers in the earlier description.

In the embodiment illustrated, the blood sampler 100 comprises a clip 2 defining a space for receiving at least part of the digit. The clip 2 is made of a static portion 25 and a movable portion 24. In this embodiment, different to the previous embodiments, a lancet holder 5 is secured to, e.g. integrally made with, the movable portion 24. The blood sampler 100 thus comprises the lancet holder 5, for receiving a lancet system 4, secured to the clip 2 at a location different from the first opening 21. In other words, the location at which blood is collected (i.e., the first opening 21) and the location at which, in use, the lancet enters the space 20, again do not overlap.

Also in this embodiment, the static portion 25 may, but does not need to, form a cup, for easy collection of a blood sample, and for guidance of the blood sample towards the first opening 21, to which a vial 3 may be attached.

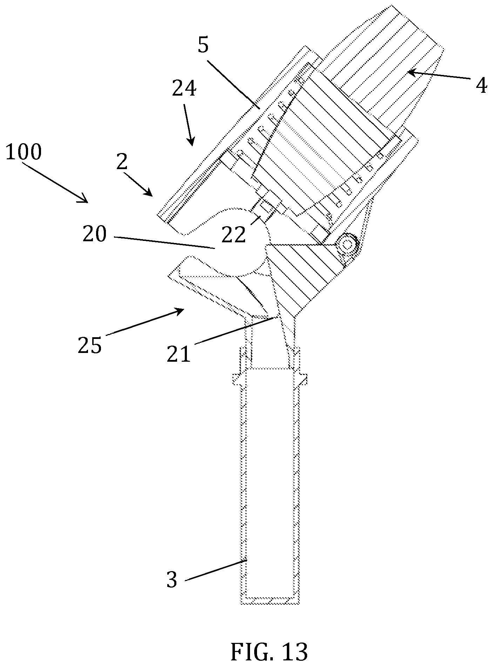

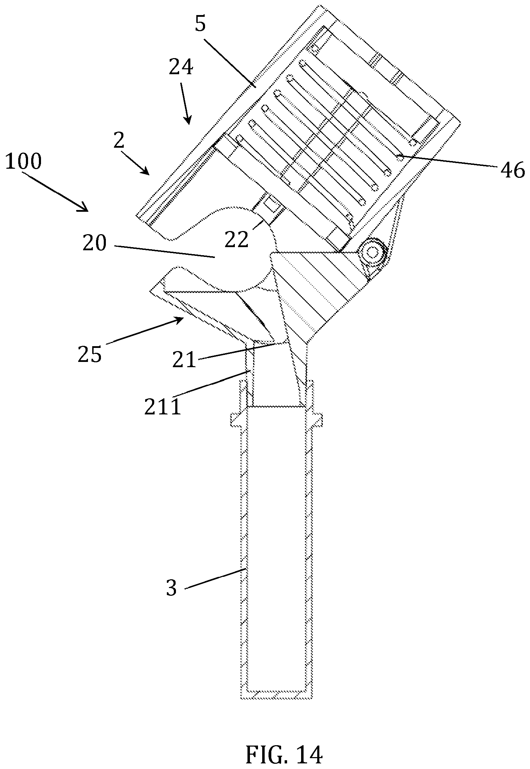

FIG. 11 and its cross-section in FIG. 13 illustrate the blood sampler 100 with a lancet system 4 introduced into the lancet holder 5. FIG. 14 illustrates a cross-section of the blood sampler 100 according to embodiments of the present invention, without the lancet system 4 being introduced therein.

As can be seen especially in FIG. 13 and FIG. 14, the blood sampler 100 may further comprise one or more springs 46 in the lancet holder 5. When pushing the lancet system 4, introduced in the lancet holder 5, the lancet system 4 may move towards the space 20, such that the lancet 43 moves through the second opening 22 and into the space 20, thereby compressing the spring 46. When the lancet system 4 is subsequently released, the spring 46 decompresses, so that the lancet system 4 is pushed away from the space 20 such that the lancet 43 moves out of the space 20.

Although particular types of lancet systems have been described above, the blood sampler according to present invention may comprise any type of lancet system as known to the person skilled in the art.

It is to be understood that although preferred embodiments, specific constructions and configurations, as well as materials, have been discussed herein for devices according to the present invention, various changes or modifications in form and detail may be made without departing from the scope of this invention.

## Claims

1. A blood sampler (1, 10, 100) for collecting a blood sample from a digit (61), preferably a finger, the blood sampler comprising:
a clip (2) defining a space (20) for receiving at least part of the digit (61), wherein the clip (2) comprises a first opening (21) for attaching a vial (3) for receiving the blood sample, and
at least one lancet holder (5) in or on the clip (2) at a location different from the first opening (21), for receiving a lancet system (4, 40) comprising a lancet (43) for lancing the at least part of the digit when present in the space (20).

2. The blood sampler (1, 10, 100) according to claim 1, wherein the at least one lancet holder (5) comprises at least one second opening (22) in the clip (2), different from the first opening (21), for allowing passage of at least a lancet of a lancet system (4) therethrough.

3. The blood sampler (1, 10, 100) according to any of the previous claims, furthermore comprising at least one lancet system (4, 40) comprising a lancet (43) that is movable between a retracted position, and a lancing position wherein the lancet (43) extends at least partially in the space (20) so as to lance the at least part of the digit when present in the space (20).

4. The blood sampler (1,10, 100) according to claim 3, wherein the at least one lancet system (4, 40) comprises a lancet casing comprising an interior and an inlet, wherein, in the retracted position, the lancet (43) is substantially located in the interior, and wherein, in the lancing position, the lancet (43) extends at least partially, through said inlet, outside of the interior.

5. The blood sampler (1, 10, 100) according to any of the previous claims, wherein the clip (2) comprises:
a static portion (25) comprising the first opening (21), and
a movable portion (24) hingeably attached to the static portion (25),
wherein the static portion (25) and/or the movable portion (24) comprises the at least one lancet holder (5).

6. The blood sampler (1, 10) according to any of the previous claims, wherein the clip (2) defines a cup (23) peripheral to the space (20), wherein the first opening (21) is located in a wall of the cup (23) and the at least one lancet holder (5) is located in or at a wall of the cup (23).

7. The blood sampler (1, 10) according to any of the previous claims, wherein an angle α between a first direction, from a centre of the space (20), in absence of the at least part of the digit (61), to the first opening (21), and a second direction, from said centre to at least one of the at least one lancet systems (4, 40), is at most 70°.

8. The blood sampler (100) according to any of claims 1 to 5, wherein the clip (2) defines a cup (23) peripheral to the space (20) and a movable portion (24), wherein the first opening (21) is located in a wall of the cup (23) and the at least one lancet holder (5) is located in or at a wall of the movable portion (24).

9. The blood sampler (1, 10, 100) according to any of the previous claims, wherein the at least one lancet holder (5) is integrally made with the clip (2).

10. The blood sampler (1, 10, 100) according to any of the previous claims, wherein the clip (2) comprises a friction element (26) for contacting the at least part of the digit (61) in the space (20).

11. The blood sampler (1, 10, 100) according to any of the previous claims, wherein the blood sampler (1) comprises means for said attaching of the vial (3) to the first opening (21) by a push-fit connection.

12. A kit of parts, comprising:
the blood sampler (1, 10, 100) according to any of the previous claims, and
a tourniquet (72) for applying a pressure to the digit (61) so as to limit a flow of blood through a vein in the digit (61).

13. The kit of parts according to claim 12, furthermore comprising at least one lancet system comprising a lancet (43).

14. The kit of parts according to any of claims 12 or 13, further comprising a vial (3), and a cap for the vial (3).

15. The kit of parts according to claim 14, wherein the vial (3) comprises a screw thread and the cap comprises a complementary screw thread.

## Patentansprüche

1. Ein Blutentnahmegerät (1, 10, 100) zum Entnehmen einer Blutprobe aus einem Finger oder einem Zeh (61), vorzugsweise einem Finger, wobei das Blutentnahmegerät Folgendes umfasst:
- eine Klemme (2), die einen Raum (20) zum Aufnehmen mindestens eines Teils des Fingers oder des Zehs (61) definiert, wobei die Klemme (2) eine erste Öffnung (21) zum Anbringen eines Fläschchens (3) zum Aufnehmen der Blutprobe umfasst, und
- mindestens ein Lanzettenhalter (5) in oder an der Klemme (2) an einer Stelle, die sich von der ersten Öffnung (21) unterscheidet, zum Aufnehmen eines Lanzettensystems (4, 40), das eine Lanzette (43) zum Anstechen des mindestens einen Teils des Fingers oder des Zehs umfasst, wenn dieser sich im Raum (20) befindet.

2. Das Blutentnahmegerät (1, 10, 100) nach Anspruch 1, wobei der mindestens eine Lanzettenhalter (5) mindestens eine zweite Öffnung (22) in der Klemme (2) aufweist, die sich von der ersten Öffnung (21) unterscheidet, um den Durchtritt mindestens einer Lanzette eines Lanzettensystems (4) durch dieselbe zu ermöglichen.

3. Das Blutentnahmegerät (1, 10, 100) nach einem der vorstehenden Ansprüche, weiter umfassend mindestens ein Lanzettensystem (4, 40), das eine Lanzette (43) umfasst, die zwischen einer zurückgezogenen Position und einer Stechposition beweglich ist, wobei sich die Lanzette (43) zumindest teilweise in den Raum (20) erstreckt, um so zumindest einen Teil des Fingers oder des Zehs, wenn dieser sich im Raum (20) befindet, anzustechen.

4. Das Blutentnahmegerät (1, 10, 100) nach Anspruch 3, wobei das mindestens eine Lanzettensystem (4, 40) ein Lanzettengehäuse umfasst, das ein Inneres und einen Einlass umfasst, wobei sich die Lanzette (43), in der zurückgezogenen Position, im Wesentlichen im Inneren befindet und wobei sich die Lanzette (43), in der Stechposition zumindest teilweise durch den Einlass aus dem Inneren heraus erstreckt.

5. Das Blutentnahmegerät (1, 10, 100) nach einem der vorstehenden Ansprüche, wobei die Klemme (2) Folgendes umfasst:
- einen statischen Abschnitt (25), der die erste Öffnung (21) umfasst, und
- einen beweglichen Abschnitt (24), der gelenkig an dem statischen Abschnitt (25) verbunden ist,
- wobei der statische Abschnitt (25) und/oder der bewegliche Abschnitt (24) den mindestens einen Lanzettenhalter (5) umfasst.

6. Das Blutentnahmegerät (1, 10) nach einem der vorstehenden Ansprüche, wobei die Klemme (2) einen Becher (23) peripher zum Raum (20) definiert, wobei sich die erste Öffnung (21) in einer Wand des Bechers (23) befindet und sich der mindestens eine Lanzettenhalter (5) in oder an einer Wand des Bechers (23) befindet.

7. Das Blutentnahmegerät (1, 10) nach einem der vorstehenden Ansprüche, wobei ein Winkel α zwischen einer ersten Richtung, von einer Mitte des Raumes (20), in Abwesenheit des mindestens einen Teils des Fingers oder des Zehs (61), zur ersten Öffnung (21), und einer zweiten Richtung, von der Mitte zu mindestens einem von dem mindestens einen Lanzettensystem (4, 40), höchstens 70° beträgt.

8. Das Blutentnahmegerät (100) nach einem der Ansprüche 1 bis 5, wobei die Klemme (2) einen Becher (23) peripher zum Raum (20) und einen beweglichen Abschnitt (24) definiert, wobei sich die erste Öffnung (21) in einer Wand des Bechers (23) befindet und sich der mindestens eine Lanzettenhalter (5) in oder an einer Wand des beweglichen Abschnitts (24) befindet.

9. Das Blutentnahmegerät (1, 10, 100) nach einem der vorstehenden Ansprüche, wobei der mindestens eine Lanzettenhalter (5) integral mit der Klemme (2) hergestellt ist.

10. Das Blutentnahmegerät (1, 10, 100) nach einem der vorstehenden Ansprüche, wobei die Klemme (2) ein Reibungselement (26) zum Kontaktieren mindestens eines Teil des Fingers oder des Zehs (61) im Raum (20) umfasst.

11. Das Blutentnahmegerät (1, 10, 100) nach einem der vorstehenden Ansprüche, wobei das Blutentnahmegerät (1) Mittel zum Anbringen des Fläschchens (3) an der ersten Öffnung (21) durch eine Steckverbindung umfasst.

12. Ein Satz von Teilen, umfassend:
- das Blutentnahmegerät (1, 10, 100) nach einem der vorstehenden Ansprüche und
- ein Tourniquet (72) zum Ausüben eines Drucks auf den Finger oder des Zehs (61), um so einen Blutfluss durch eine Vene in dem Finger oder dem Zeh (61) zu begrenzen.

13. Der Satz von Teilen nach Anspruch 12, weiter umfassend mindestens ein Lanzettensystem, das eine Lanzette (43) umfasst.

14. Der Satz von Teilen nach einem der Ansprüche 12 oder 13, weiter umfassend ein Fläschchen (3) und eine Kappe für das Fläschchen (3).

15. Der Satz von Teilen nach Anspruch 14, wobei das Fläschchen (3) ein Schraubgewinde umfasst und die Kappe ein komplementäres Schraubgewinde umfasst.

## Revendications

1. Un échantillonneur de sang (1, 10, 100) pour collecter <un échantillon de sang à partir d'un doigt (61), de préférence un doigt de la main, ledit échantillonneur de sang comprenant :
une pince (2) définissant un espace (20) pour recevoir au moins une partie du doigt (61), dans laquelle ladite pince (2) comprend une première ouverture (21) pour fixer un flacon (3) destiné à recevoir l'échantillon de sang, et
au moins un support de lancette (5) dans ou sur ladite pince (2) à un emplacement différent de la première ouverture (21), pour recevoir un système de lancette (4, 40) comprenant une lancette (43) pour piquer au moins une partie du doigt lorsqu'elle est présente dans l'espace (20).

2. L'échantillonneur de sang (1, 10, 100) selon la revendication 1, dans lequel ledit au moins un support de lancette (5) comprend au moins une deuxième ouverture (22) dans ladite pince (2), différente de la première ouverture (21), pour permettre le passage d'au moins une lancette d'un système de lancette (4) à travers celle-ci.

3. L'échantillonneur de sang (1, 10, 100) selon l'une quelconque des revendications précédentes, comprenant en outre au moins un système de lancette (4, 40) comprenant une lancette (43) qui est mobile entre une position rétractée et une position de piqûre dans laquelle ladite lancette (43) s'étend au moins partiellement dans l'espace (20) de manière à piquer au moins une partie du doigt lorsqu'elle est présente dans l'espace (20).

4. L'échantillonneur de sang (1, 10, 100) selon la revendication 3, dans lequel ledit au moins un système de lancette (4, 40) comprend un boîtier de lancette comprenant un intérieur et une entrée, dans lequel, en position rétractée, ladite lancette (43) est substantiellement située à l'intérieur, et dans lequel, en position de piqûre, ladite lancette (43) s'étend au moins partiellement, à travers ladite entrée, à l'extérieur de l'intérieur.

5. L'échantillonneur de sang (1, 10, 100) selon l'une quelconque des revendications précédentes, dans lequel ladite pince (2) comprend :
une partie statique (25) comprenant la première ouverture (21), et
une partie mobile (24) fixée de manière articulée à ladite partie statique (25),
dans laquelle ladite partie statique (25) et/ou ladite partie mobile (24) comprend ledit au moins un support de lancette (5).

6. L'échantillonneur de sang (1, 10) selon l'une quelconque des revendications précédentes, dans lequel ladite pince (2) définit une coupelle (23) périphérique à l'espace (20), dans laquelle la première ouverture (21) est située dans une paroi de ladite coupelle (23) et ledit au moins un support de lancette (5) est situé dans ou sur une paroi de ladite coupelle (23).

7. L'échantillonneur de sang (1, 10) selon l'une quelconque des revendications précédentes, dans lequel un angle α entre une première direction, depuis un centre de l'espace (20), en l'absence d'au moins une partie du doigt (61), vers la première ouverture (21), et une deuxième direction, depuis ledit centre vers au moins un desdits au moins un système de lancette (4, 40), est d'au plus 70°.

8. L'échantillonneur de sang (100) selon l'une quelconque des revendications 1 à 5, dans lequel ladite pince (2) définit une coupelle (23) périphérique à l'espace (20) et une partie mobile (24), dans laquelle la première ouverture (21) est située dans une paroi de ladite coupelle (23) et ledit au moins un support de lancette (5) est situé dans ou sur une paroi de ladite partie mobile (24).

9. L'échantillonneur de sang (1, 10, 100) selon l'une quelconque des revendications précédentes, dans lequel ledit au moins un support de lancette (5) est fabriqué de manière intégrée avec ladite pince (2).

10. L'échantillonneur de sang (1, 10, 100) selon l'une quelconque des revendications précédentes, dans lequel ladite pince (2) comprend un élément de friction (26) pour entrer en contact avec au moins une partie du doigt (61) dans l'espace (20).

11. L'échantillonneur de sang (1, 10, 100) selon l'une quelconque des revendications précédentes, dans lequel ledit échantillonneur de sang (1) comprend des moyens pour ladite fixation du flacon (3) à la première ouverture (21) par une connexion à emboîtement.

12. Un kit de pièces, comprenant :
ledit échantillonneur de sang (1, 10, 100) selon l'une quelconque des revendications précédentes, et
un garrot (72) pour appliquer une pression sur le doigt (61) de manière à limiter un flux de sang à travers une veine dans le doigt (61).

13. Le kit de pièces selon la revendication 12, comprenant en outre au moins un système de lancette comprenant une lancette (43).

14. Le kit de pièces selon l'une quelconque des revendications 12 ou 13, comprenant en outre un flacon (3), et un bouchon pour ledit flacon (3).

15. Le kit de pièces selon la revendication 14, dans lequel ledit flacon (3) comprend un filetage et ledit bouchon comprend un filetage complémentaire.
